# EUROPÄISCHE PATENTSCHRIFT

(11) **EP 0 972 203 B1**
(45) Veröffentlichungstag und Bekanntmachung des Hinweises auf die Patenterteilung: **25.02.2004**
(21) Anmeldenummer: 98909216.8
(22) Anmeldetag: 26.03.1998
(51) Int. Cl.: G01N 33/86

(54) **REAGENS ZUR BESTIMMUNG DER aPTT**
REAGENT FOR DETERMINING aPTT
REACTIF POUR DETERMINER LE TEMPS DE CEPHALINE ACTIVE

(30) Priorität: 27.03.1997 AT 53397
(43) Veröffentlichungstag der Anmeldung: 19.01.2000
(73) Patentinhaber: Baxter Aktiengesellschaft, 1220 Wien (AT)
(72) Erfinder: MORITZ, Berta, A-1180 Wien (AT); VARADI, Katalin, A-1230 Wien (AT); LANG, Hartmut, A-2560 Grillenberg (AT); SCHWARZ, Hans-Peter, A-1180 Wien (AT)
(74) Vertreter: Alge, Daniel, Mag. Dr. rer.nat.
(86) Internationale Anmeldenummer: PCT/AT1998/000082
(87) Internationale Veröffentlichungsnummer: WO 1998/044352

(56) Entgegenhaltungen:
- WO-A-97/20066
- US-A- 5 506 146

## Beschreibung

Die Erfindung betrifft ein Reagens zur Bestimmung der aPTT sowie ein Verfahren zur Bestimmung der APC-Cofaktor-Aktivität bzw. eines APC-Substrates in einer Probe.

Die aktivierte partielle Thromboplastinzeit (aPTT) einer Plasmaprobe dient als "screening"-Test zur globalen Erfassung der Aktivität von Gerinnungsfaktoren der intrinsischen Gerinnung. Störungen im Gerinnungsablauf bedingen Gerinnungszeiten, die außerhalb des Normalbereiches liegen. Die Verlängerung der aPTT, wie sie bei der Hämophilie A auftritt, manifestiert z.B. einen Mangel an Gerinnungsfaktor VIII. Zusätzlich wird der aPTT-Test häufig zur Überwachung einer Heparintherapie eingesetzt.

Ein Reagens zur Bestimmung der aPTT enthält einen Aktivator, um die Kaskade der intrinsischen Gerinnung bis zur Bildung von aktiviertem Faktor IX zu initiieren: Desweiteren werden zur Aktivierung von Faktor X und Prothrombin Phospholipide benötigt, weshalb diese ebenfalls in einem aPTT-Reagens enthalten sind. Das Reagens wird mit einer Plasmaprobe in Kontakt gebracht, worauf nach einer definierten, möglichst kurzen Aktivierungszeit Calcium zugesetzt wird und die Zeit bis zur Bildung eines Fibrinclots gemessen wird.

An das Testsystem der aPTT werden eine Vielzahl von Anforderungen gestellt. Für Routineuntersuchungen ist die einfache Handhabung eines aPTT-Reagens unbedingte Voraussetzung. Ebenso muß die Stabilität der Inhaltsstoffe sehr hoch sein, um eine gleichbleibende Gerinnungszeit über einen langen Zeitraum hinweg zu gewährleisten. Dies ermöglicht auch den kostengünstigen Einsatz des Reagens. Auch ist für die aPTT als Globaltest eine ausgewogene Sensitivität gegenüber allen zu erfassenden Parametern erforderlich.

Übliche Reagentien zur Bestimmung der aPTT enthalten als Initiator der intrinsischen Gerinnung einen oberflächenaktiven Feststoff. Durch die Verwendung einer oberflächenaktiven Matrix wird zuerst Faktor XII aktiviert, vergleichbar mit den physiologischen Bedingungen. Als Feststoffaktivatoren werden etwa Kaolin, Celit und Glas eingesetzt. Kaolin ist beispielsweise in handelsüblichen Reagentien zur Bestimmung der aPTT als Suspension enthalten (Pathromtin®, Fa. Behringwerke (1990) oder PTT-Reagens, Fa. Boehringer Mannheim (1986)). Als Phospholipid wird ein lyophilisierter Lipidextrakt aus Humanplazenten bzw. lyophilisiertes Kephalin eingesetzt. Die Phospholipide werden jedoch erst vor der Durchführung der Bestimmung mit Kaolin zu einem gebrauchsfertigen Reagens gemischt. Die getrennte Lagerung von Kaolin und Phospholipiden ist vor allem aus Stabilitätsgründen erforderlich.

Bei der Lyophilisierung von Lipiden mit Kaolin würden Lipide großteils inaktiviert werden. So tritt etwa bei der Herstellung von PTT-IMMUNO (Fa. Immuno) bei der gemeinsamen Lyophilisierung von Kaolin und Phospholipiden (extrahiert aus Schweinehirn) ein Verlust von bis zu 50 % der Phospholipidaktivität auf.

Als Alternative zu Feststoffaktivatoren können auch flüssige Aktivatoren, wie Ellagsäure und ihre Derivate, oder gelöste Sulfatide eingesetzt werden. Die Empfindlichkeit eines Flüssigaktivatoren enthaltenden Tests gegenüber Heparin ist jedoch verschlechtert, weshalb diverse Zusätze zur Empfindlichkeitsverbesserung vorgeschlagen werden. Gemäß der WO 91/16453 enthält ein heparinempfindliches Reagens auf Basis von Ellagsaure zusätzlich Dextransulfat.

Ein Nachteil von Kaolin bzw. von Feststoffaktivatoren im allgemeinen und Ellagsäure bzw. -derivaten ist die lange Aktivierungszeit der ersten Phase der intrinsischen Gerinnung, wodurch eine relativ lange Inkubationszeit von mindestens 4 min zur Aktivierung notwendig ist. Ein weiterer Nachteil besteht in der relativ langen Gerinnungszeit in Gegenwart von Kaolin. Eine höhere Kaolinkonzentration im Reagens kann jedoch die Aktivierungszeit verkürzen. Kaolin, das sich aus der Suspension absetzt, kann aber zu einer verlängerten Gerinnungszeit führen bzw. Fehlergebnisse bei der Ermittlung des Gerinnungsendpunktes mittels gebräuchlicher Koagulometer hervorrufen. Es muß daher getrachtet werden, die Konzentrationen an Feststoffaktivator möglichst gering zu halten.

Um kurze Aktivierungszeiten und auch eine hohe Empfindlichkeit gegenüber Heparin oder Einzelfaktoren der Gerinnungskaskade zu erzielen, wird beispielsweise in der EP 0 570 356 als Aktivator ein Gemisch aus Sulfatiden und einem Feststoffaktivator, beispielsweise Kaolin, vorgeschlagen.

Obwohl mittels aPTT die Funktionsfähigkeit der intrinsischen Gerinnungsfaktoren bestimmt wird, haben auch Inhibitorsysteme, die gegen diese Faktoren wirken, beispielsweise Proteine des Protein C-Pathways, einen Einfluß und sind damit indirekt bestimmbar.

Protein C ist ein Vitamin K-abhängiges Glycoprotein, das in der Leber synthetisiert wird und im Plasma als inaktives Zymogen in einer Konzentration von etwa 4 µg/ml zirkuliert. Dieses Protein wird durch den Thrombin-Thrombomodulinkomplex an der Oberfläche der Gefäßwand (Endothelium) in eine aktive Serinprotease (aktiviertes Protein C, APC) umgewandelt. Protein C kann auch durch nicht-physiologische Enzyme, wie z.B. Schlangengift von Agkistrodon contortrix oder Serinproteasen, wie Trypsin oder Plasmin, aktiviert werden.

APC hat eine Antikoagulationswirkung, da es Faktor Va und Faktor VIIIa proteolytisch abbauen kann. Faktor Va ist ein Cofaktor für die Faktor Xa-induzierte Aktivierung von Prothrombin zu Thrombin. Faktor VIIIa ist der Cofaktor der Umwandlung von Faktor X zu Faktor Xa. Daher stellt die Aktivierung von Protein C in vivo zur Bildung von APC eine negative Feedback-Reaktion bei der Erzeugung von Thrombin auf dem Weg über den Abbau der Faktoren Va und VIIIa dar.

Zur Entwicklung der optimalen APC-Aktivität sind Cofaktoren, wie beispielsweise Protein S, notwendig. Im Plasma ist Protein S in verschiedenen Formen vorhanden. Zu diesen Formen gehört eine freie, aktive Form und eine inaktive Form, die mit dem C4b-binding-Protein nicht-kovalent komplexiert ist. In einem Protein S-verarmten Plasma kann APC seine normale Aktivität nicht ausüben. Nach Zugabe von Protein S erlangt das APC seine normale Aktivität wieder.

Aktiviertes Protein C (APC) erhöht die aPTT des Plasmas in Abhängigkeit von der Dosis. Eine verringerte APC-Empfindlichkeit (= APC-Resistenz) liegt vor, wenn die Zugabe von APC zur Probe zu keiner oder geringeren Verlängerung der Gerinnungszeit führt (siehe Dahlbäck et al., PNAS 96, 1004-8, (1993)).

Für die APC-Resistenz werden verschiedene Ursachen diskutiert. Bertina et al. (Nature, 369, S. 64-67, (1994)) fanden eine der Ursachen in einem Defekt im Faktor V-Molekül, nämlich in einer Punktmutation an der Aminosäureposition 506 (Faktor V-Leiden). Das Vorliegen einer derartigen Mutation führt dazu, daß der aktivierte Faktor V-Leiden von APC nicht richtig abgebaut werden kann, damit wird die Reaktion von APC auf die Gerinnungskaskade vermindert.

Der normale Faktor V wirkt auch als Cofaktor von APC in Synergie mit Protein S im Abbau von Faktor VIII. Auch diese Cofaktor-Aktivität geht bei Vorliegen einer Mutation im Faktor V-Molekül teilweise verloren (Varadi K. et al., Thrombosis and Haemostasis, 76, 2, S. 208-214 (1996)). Eine verminderte APC-Empfindlichkeit kann aber auch in einem Protein S-Mangel oder einem abnormen Faktor VIII-Molekül begründet sein.

Aus dem Stand der Technik sind auch verschiedene Verfahren zur Diagnose von Blutgerinnungsstörungen bekannt. Gemäß der EP-0 608 235 wird ein Testkit eingesetzt, welcher als Komponenten u.a. APC (oder Protein C, zusammen mit gebräuchlichen Reagentien, die Protein in APC überführen) aufweist. Weiters umfaßt der dort beschriebene Testkit ein aPTT-Reagens, das einen Kontakt-Aktivator des intrinsischen Weges umfaßt, sowie eine Phospholipid-Komponente und eine Calciumsalz-Komponente in getrennten Behältern oder als Gemisch von mindestens zwei der Komponenten. Jedoch wird das APC nicht zusammen mit anderen Komponenten in einem Reagens zur Verfügung gestellt, sondern getrennt unmittelbar vor der aPTT-Bestimmung zugegeben, womit mehrere Pipettierungsschritte vor dem eigentlichen Test erforderlich sind.

Ein Vergleich mit dem Test ohne Zugabe von APC ermöglicht die Bestimmung einer APC-Resistenz. Das Verfahren ist jedoch nicht zur Bestimmung von Plasmen von OAK-Patienten verwendbar und auch nicht bei Vorliegen verschiedener Mangelzustände, die zu einer verlängerten aPTT führen.

In der EP-0 656 424 ist ein Test zur Bestimmung der Empfindlichkeit gegenüber APC auf Basis eines chromogenen Substrats beschrieben, wobei die Empfindlichkeit einer Testprobe gegenüber APC durch die relative Inhibition der Faktor X-Umwandlung im Vergleich zu einer Kontrolle bestimmt wird. Die jeweiligen Testreagentien werden dabei unmittelbar vor der Bestimmung mit APC durch Pipettierungsschritte gemischt.

Die Evaluierung der Testergebnisse erfolgt bei beiden Verfahren durch zweimalige Durchführung des jeweiligen Tests, einmal in Anwesenheit von APC und einmal in Abwesenheit von APC, und anschließende Bildung des Verhältniswertes.

Ein Beispiel zur Bestimmung der Protein S-Aktivität wird in der EP-0 445 626-A beschrieben. In diesem Test wird in einem Reagens zur funktionellen Protein S-Bestimmung, welches APC, einen Aktivator des exogenen oder endogenen Weges der Gerinnung Phospholipide, Calciumionen, gegebenenfalls ein chromogenes Substrat für Thrombin, sowie eine Heparin-neutralisierende Substanz enthält, das Verhältnis der Aktivitäten des APC und des Aktivators so eingestellt, daß eine Gerinnungszeit in Abwesenheit von Protein S von mindestens 50 sek gewährleistet ist.

Bei der Routineuntersuchung von aPTT-Werten in Testproben, insbesondere zur Bestimmung der APC-Cofaktor-Aktivität oder zur Bestimmung der Aktivität eines APC-Substrates, ist es notwendig, die Zahl an Pipettierungsschritten möglichst gering zu halten, speziell um einerseits eine schnelle Durchführung der Tests zu sichern und andererseits die Gefahr von Fehlern bzw. Konzentrationsabweichungen zu vermeiden.

Die vorliegende Erfindung stellt sich zur Aufgabe, ein Reagens und ein Verfahren zur Verwendung bzw. Messung der aPTT zur Verfügung zu stellen, mit welchen APC-Cofaktor-Aktivität bzw. die Aktivität eines APC-Substrates in einer Probe routinemäßig auch in klinischen Reihenversuchen durchgeführt werden können, insbesondere in welchen auch Plasmen oral antikoagulierter Personen bestimmbar sind, ohne beispielsweise zusätzliche Verdünnungsschritte oder Kontrollmessungen durchführen zu müssen.

Die gestellte Aufgabe wird durch ein Reagens zur Bestimmung der aPTT, umfassend zumindest ein Gerinnungsprotein, Phospholipide und einen Aktivator der intrinsischen Gerinnung in co-lyophilisierter Form, gelöst.

Überraschenderweise kann ein derartiges Reagens in lyophilisierter Form zur Verfügung gestellt werden, ohne daß dabei die Aktivität oder Funktionalität der enthaltenen Komponenten in Bezug auf das Testverfahren negativ beeinflußt wird. Mit dem erfindungsgemäßen Reagens kann vor allem bei klinischen Reihenuntersuchungen zumindest ein Pipettierungsschritt "eingespart" werden, was bei solchen Tests eine erhebliche wirtschaftliche, zeitliche und verfahrenstechnische Erleichterung mit sich bringt.

Darüberhinaus wird mit der Anwesenheit von einem oder mehreren Gerinnungsproteinen eine aussagekräftige Endpunktbestimmung der aPTT möglich.

Durch das Vorhandensein von zumindest einem Gerinnungsprotein im Reagens ist die aPTT dann von der Anwesenheit des in der Probe natürlicherweise vorkommenden Proteins unabhängig. Dies ist beispielsweise bei Faktor V oder Faktor VIII von Bedeutung, dessen Aktivität bei der Lagerung von Plasmen relativ stark abnimmt. Verwendet man nun ein erfindungsgemäßes Reagens, enthaltend eine standardisierte Menge an Faktor V, so ist die aPTT unabhängig vom aktuellen Faktor V-Spiegel der zu testenden Plasmen, und eine veränderte aPTT läßt sich dann eindeutig einem Mangel an einem anderen Protein zuordnen.

Weiters kann mit der vorliegenden Erfindung auch in den meisten Fällen eine Verdünnung der Testproben mit einem Mangelplasma entfallen, weil im Reagens die Faktoren der intrinsischen Gerinnung außer dem zu bestimmenden Faktor im Überschuß zugegeben werden können und auf diese Weise die Spezifität des Tests für den zu bestimmenden Faktor gewährleistet ist.

Da alle benötigten Gerinnungsfaktoren bis auf den zu untersuchenden im Reagens enthalten sein können, ist eine Einzelfaktorbestimmung in Anlehnung an die 1-Stufen-Methode möglich.

Das Gerinnungsprotein ist vorzugsweise ausgewählt aus der Gruppe der Vitamin K-abhängigen Faktoren, der intrinsischen Gerinnungsfaktoren (wobei sowohl die Vitamin K-abhängigen Faktoren als auch die intrinsischen Gerinnungsfaktoren gegebenenfalls aktiviert sein können), der Prothrombinkomplex-Faktoren, der intrinsischen Cofaktoren und der Cofaktoren der Gerinnung.

Die Gerinnungsproteine sind daher vorzugsweise ausgewählt aus der Gruppe Faktor II, Faktor VII, Faktor IX, Faktor X, Protein C, Protein Z und Protein S, gegebenenfalls in aktivierter Form, oder Kombinationen derselben.

In einer besonders bevorzugten Ausführungsform sind im Reagens die Faktoren II, IX und X und gegebenenfalls Protein S enthalten. Dadurch sind insbesondere Plasmen oral antikoagulierter Patienten (OAK-Plasmen) mittels aPTT bestimmbar, insbesondere ist das Vorliegen einer APC-Resistenz feststellbar, da die Vitamin K-abhängigen Faktoren ja im Überschuß vorliegen. Im Reagens können aktivierte Faktoren neben nicht aktivierten Faktoren vorliegen. In einer bevorzugten Ausführungsform liegt im Reagens jedoch kein aktivierter Faktor II vor. Dadurch sind speziell Erleichterungen der Messungen im Hinblick auf die Verdünnung mit Mangelplasmen erzielbar.

Eine besonders bevorzugte Ausführungsform des erfindungsgemäßen Reagens umfaßt Faktor XI oder Faktor XII, gegebenenfalls in aktivierter Form. Das Reagens kann auch Kontaktfaktoren, wie HMW-Kininogen und Prekallikrein, enthalten.

Bei einer anderen bevorzugten Ausführungsform des erfindungsgemäßen Reagens wird als Gerinnungsprotein Faktor V oder Faktor VIII verwendet.

Die Gerinnungsproteine liegen im Reagens bevorzugterweise in gereinigter Form vor. Die Gerinnungsproteine können aus Plasma oder Plasmafraktionen stammen, sie können aber auch rekombinant hergestellt werden. Die Reinigung selbst erfolgt mittels für diese Proteine gängigen Methoden, vorzugsweise kommen chromatographische Verfahren zum Einsatz.

Bei einer weiteren bevorzugten Ausführungsform enthält das erfindungsgemäße Reagens aktiviertes Protein C als Gerinnungsprotein, welches nach Rekonstitution in einer Aktivität von mindestens 0,05 E/ml Reagens vorliegt, vorzugsweise in einem Bereich zwischen 0,05 bis 2 E/ml, wobei ein Bereich zwischen 0,1 bis 1 E/ml, insbesondere 0,2 bis 0,6 E/ml, besonders bevorzugt ist.

Ein besonders vorteilhaftes Reagens enthält aktiviertes Protein C und die Faktoren II, IX und X, sowie gegebenenfalls Faktor VII und/oder Protein S.

Das Gerinnungsprotein kann erfindungsgemäß in einer standardisierten Mischung bestehend aus Faktor II, Faktor IX und Faktor X in Konzentrationen von jeweils 0,05 bis 2 E/ml im Reagens enthalten sein.

Die weiteren Faktoren VII und/oder Protein S liegen dann bevorzugterweise in einer Konzentration zwischen 0,1 bis 2 E/ml, insbesondere zwischen 0,2 und 1 E/ml, im Reagens vor.

Als Aktivatoren kommen feste oder flüssige Komponenten zum Einsatz, auch Kombinationen fest-flüssig sind möglich.

Der Aktivator der intrinsischen Gerinnung im erfindungsgemäßen Reagens ist vorteilhafterweise ausgewählt aus der Gruppe Ellagsäure, Celit, Silizium-hältige Verbindungen und Sulfatide oder Derivaten bzw. Kombinationen dieser Aktivatoren. Besonders bevorzugt wird eine Kombination aus Kaolin und Sulfatid (DAPTTIN, Fa. IMMUNO AG, Wien) verwendet.

Die Phospholipide, welche im erfindungsgemäßen Reagens zum Einsatz kommen, liegen vorzugsweise in reiner Form in einer standardisierten Mischung vor, wobei dabei Verhältnisse der Phospholipide in der Mischung von 5 bis 50 % Phosphatidylserin, 15 bis 60 % Phosphatidylcholin, 15 bis 60 % Phosphatidylethanolamin; 0 bis 20 % Cholesterin und 5 bis 20 % Phosphatidylsäure (die Prozente sind bezogen auf den Gesamtphospholipidgehalt) vorgesehen werden.

Als standardisierte Mischung werden z.B. 0,2 mg Phosphatidylserin, 0,85 mg Phosphatidylcholin, 0,65 mg Phosphatidylethanolamin, 0,15 mg Cholesterin und 0,15 mg Phosphatidinsäure pro ml eventuell verdünnt eingesetzt.

Eine bevorzugte Ausführungsform des Reagens ist dadurch gekennzeichnet, daß es weiters Oxidationshemmer und Empfindlichkeitsverbesserer enthält. Als Oxidationshemmer zur Erhöhung der Lagerstabilität des Reagens im lyophilisierten oder rekonstituierten Zustand werden beispielsweise Cystein, Harnsäure, Vitamin C oder Vitamin E eingesetzt. Zusätze, wie Alkalisalze, eventuell gemeinsam mit Aminosäuren, erhöhen die Sensitivität des Tests gegenüber Einzelfaktoren. Es hat sich gezeigt, daß sich ein Reagens, enthaltend Cäsiumchlorid (5 bis 25 mg/ml) und Arginin (2,5 bis 20 mg/ml), vorzugsweise 16,8 mg CsCl/ml und 10,5 mg Arginin/ml, nach entsprechender Verdünnung besonders gut zur Bestimmung der aPTT einer Plasmaprobe mit einem Mangel an Einzelfaktoren (Faktor VIII) eignet.

Das erfindungsgemäße Reagens ist vorzugsweise frei von Calciumionen, wodurch seine Stabilität verbessert wird.

Das erfindungsgemäße Reagens kann weiters eine Heparin-neutralisierende Substanz, vorzugsweise ausgewählt aus der Gruppe Polybren, Protamin-Salzen und Heparinase, sowie einen oder mehrere Stabilisatoren, vorzugsweise ausgewählt aus der Gruppe Albumin, Puffersubstanz, Aminosäure, Zucker und Gelatine, enthalten.

Weiters kann das erfindungsgemäße Reagens ein chromogenes Substrat enthalten, mit dem gegebenenfalls die Gerinnungszeit photometrisch durch Farbentwicklung bestimmbar ist. Die Erfassung des Endprodukts ist aber auch durch photometrische Bestimmung der Veränderung der Lichtdurchlässigkeit möglich.

Eine besonders bevorzugte Ausführungsform des erfindungsgemäßen Reagens zur chromogenen Bestimmung der aPTT weist eine Clotbildungs-hemmende Substanz, vorzugsweise ein Tetrapeptid, wie z.B. AcOH-Gly-Pro-Arg-Pro-OH, auf.

Das erfindungsgemäße Reagens ist auf Grund seiner Zusammensetzung bzw. auf Grund der Reinheit der eingesetzten Substanzen bevorzugterweise nach Rekonstitution als Lösung bei Raumtemperatur über einen Zeitraum von mindestens 3 Stunden, vorzugsweise mindestens 8 Stunden, am meisten bevorzugt mindestens 24 Stunden, lange stabil.

Gemäß einem weiteren Aspekt betrifft die vorliegende Erfindung ein Verfahren zur Bestimmung der APC-Resistenz; der APC-Cofaktor-Aktivität bzw. eines APC-Substrates in einer Probe, welches dadurch gekennzeichnet ist, daß die Probe mit dem (rekonstituierten) erfindungsgemäßen Reagens in Gegenwart von Calciumionen in Kontakt gebracht, die Blutgerinnung ausgelöst und das Ausmaß der Gerinnung bestimmt wird. Das erhaltene Ergebnis hat dann für den Fachmann durch Vergleich mit dem bereits bekannten und vorher bestimmten Normalbereich unmittelbare Aussagekraft. Damit kann auf die Bezugnahme auf den gleichen Test ohne APC-Zusatz verzichtet werden, was wiederum die Testdurchführung vereinfacht.

Mit dem erfindungsgemäßen Verfahren kann bevorzugterweise die APC-Cofaktor-Aktivität des Faktor V und/oder des Protein S bestimmt werden.

Insbesondere kann mit dem erfindungsgemäßen Verfahren in einfacher Weise ein Mangel und/oder eine funktionelle Störung eines APC-Cofaktors oder eines APC-Substrats bestimmt werden, indem ein APC-hältiges Reagens verwendet wird. Durch Variation weiterer Zusätze, wie Zugabe von Faktor V und/oder Protein S, zu dem Reagens kann eine differenzierte Diagnose der jeweiligen Mangelzustände erfolgen.

Ebenfalls kann durch das erfindungsgemäße Verfahren das Vorliegen einer Faktor VIII-Anomalie qualitativ und quantitativ bestimmt werden. Ein für diese Zwecke geeignetes.Reagens enthält insbesondere APC, Faktor V, Protein S und gegebenenfalls andere intrinsische Vitamin K-abhängige Faktoren.

Eine bevorzugte Ausführungsform des erfindungsgemäßen Verfahrens besteht darin, einer Probe, die auch von OAK-Patienten stammen kann, ein Reagens umfassend APC, Faktor II, IX und X sowie gegebenenfalls Faktor VII und/oder Protein S zuzugeben und anschließend die Gerinnungszeit zu messen.

Bevorzugterweise wird der Probe ein Faktor V-Mangelplasma, Protein S-Mangelplasma oder Faktor V/Protein S-Mangelplasma zugegeben. Durch den Einsatz des erfindungsgemäßen Reagens kann dann über die Funktion von Faktor V und/oder Protein S der Probe eine aussagekräftige Information erhalten werden.

Das Ausmaß der Gerinnung kann durch alle möglichen gängigen Bestimmungsverfahren gemessen werden, vorzugsweise jedoch unter Verwendung eines Koagulometers.

Das erfindungsgemäße Verfahren wird vorzugsweise derart durchgeführt, daß zunächst ein Normalbereich für die APC-Empfindlichkeit, für den zu bestimmenden APC-Cofaktor oder für das APC-Substrat ausschließlich in Anwesenheit von APC ermittelt wird und die Probe anschließend erfindungsgemäß vermessen wird.

Gemäß einem weiteren Aspekt betrifft die vorliegende Erfindung die Verwendung des erfindungsgemäßen Reagens zur Bestimmung der APC-Resistenz, eines Mangels und/oder einer funktionellen Störung eines APC-Cofaktors oder eines APC-Substrats und/oder zur Bestimmung einer Faktor VIII-Anomalie.

Schließlich betrifft die vorliegende Erfindung auch ein Set zur Durchführung des erfindungsgemäßen Verfahrens, enthaltend
(i) ein erfindungsgemäßes Reagens und
(ii) eine Kontrollprobe bzw. einen Kalibrator.

Die vorliegende Erfindung wird durch nachstehende Beispiele und der Zeichnungsfigur näher erläutert, ohne jedoch auf diese beschränkt zu sein.

Es zeigt:

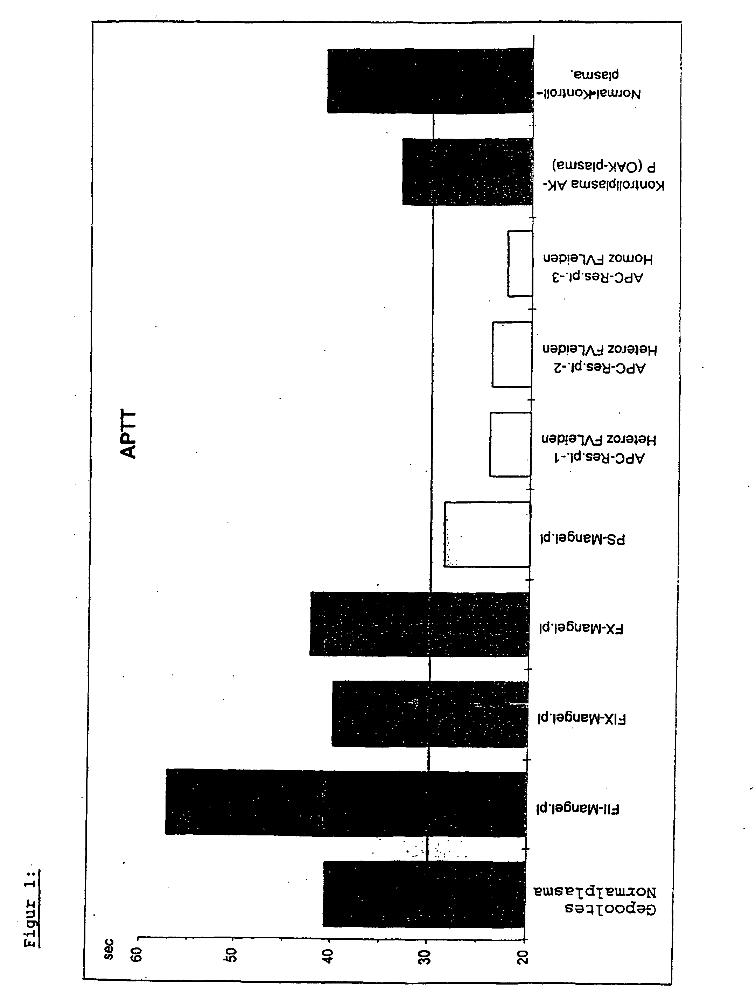

- Fig. 1:: die Bestimmung der aPTT mit dem erfindungsgemäßen Reagens

### Beispiele:

### Beispiel 1: APC-aPTT-Reagens

**Zusammensetzung des Reagens:** DAPTTIN (erhältlich von Fa. IMMUNO, AT) enthaltend Kaolin und Sulfatid als Oberflächenaktivator und eine Phospholipidmischung aus fünf hochreinen Komponenten in einem HEPES/Glycin-Puffer, pH 7,0, werden in 9,5 ml Aqua dest. gelöst und mit 0,5 ml einer Lösung versetzt, die APC (0,5 E APC/ml, erhältlich von Fa. IMMUNO, AT) enthält. Anschließend wird das Reagens lyophilisiert. Vor der Verwendung wird das Lyophilisat mit 10 ml Aqua dest. gelöst und ist dann für mindestens 8 Stunden bei Raumtemperatur stabil.

### Beispiel 2 :

### Einfluß der Zugabe von Faktoren II, IX und X auf die aPTT-Werte von verschiedenen Plasmaproben

Die Gerinnungszeit von verschiedenen Plasmaproben wird mit drei unterschiedlichen Verfahren (A, B und C (erfindungsgemäß)) gemessen.

Die eingesetzten Kontroll- bzw. Mangelplasmen sind Produkte der Firma IMMUNO AG. Die gereinigten Faktoren IX, X und II wurden von den Enzyme Research Laboratories (USA) erhalten. Das gepoolte Normalplasma (fresh-frozen) wurde von den George King Laboratories (USA) erhalten.

### Verfahren A: Kontroll-aPTT:

100 µl Plasmaprobe werden mit 100 µl DAPTTIN für 2 min bei 37°C im Koagulometer vorinkubiert Die Gerinnungszeitmessung wird mit 100 µl 25 mM Calciumchlorid gestartet.

### Verfahren B: "Klassische Methode" (aPTT-Messung in Gegenwart von APC, jedoch ohne weitere Gerinnungsproteinzugaben):

100 µl Plasmaprobe werden mit 100 µl DAPTTIN und 0,2 IE/ml APC (Gesamtvolumen: 250 µl in HNaBSA-haltigem Puffer (25 mM HEPES, 175 mM Natriumchlorid, pH 7,4 + 1 mg/ml BSA)) für 2 min bei 37°C im Koagulometer (KC 10; Fa. Amelung) vorinkubiert. Die Gerinnungszeitmessung wird mit 50 µl 50 mM Calciumchlorid gestartet.

### Verfahren C: Erfindungsgemäßes Verfahren (die aPTT wird in Gegenwart von APC, Faktor II, Faktor X und Faktor IX gemessen; derzeit nach Ansicht der Anmelderin der beste Weg zur Ausführung der Erfindung):

50 µl unverdünnte Plasmaprobe werden mit 150 µl Reagens, bestehend aus DAPTTIN, APC, Faktor II, Faktor X und Faktor IX, in einer finalen Konzentration von jeweils 0,2 IE/ml für 2 min bei 37°C im Koagulometer vorinkubiert. Die Gerinnungszeitmessung wird mit 100 µl 25 mM Calciumchlorid gestartet und mittels Koagulometer bestimmt. Das Reagens kann in lyophilisiertem Zustand gelagert werden und ist nach Rekonstitution gebrauchsfertig.

Die Ergebnisse sind in Tabelle I aufgelistet, wobei der Normalbereich von Werten von Pläsmaproben mit normalen Aktivitäten aller Faktoren (Enzym, Substrat, Cofaktor) des Protein C-Pathways bestimmt wird.

**TABELLE I**

| Plasmen | A | B | C |
|---|---|---|---|
| | | aPTT, sek | |
| | ohne APC | + APC | + APC +FII,FX,FIX |
| gepooltes Normalplasma | 37,4 | 99,5 | 40,6 |
| FII-Mangelplasma | 133,8 | >1000 | 57,3 |
| FV-Mangelplasma | 157,3 | 338,9 | 151,6 |
| FVIII-Mangelplasma | 98,6 | >1000 | 745,7 |
| FIX-Mangelplasma | 124,7 | >1000 | 40,0 |
| FX-Mangelplasma | 124,0 | >1000 | 42,4 |
| PS-Mangelplasma | 40,4 | 64,8 | 28,7 |
| APC-Res.pl.-1 Heteroz FV-Leiden | 32,9 | 59,0 | 24,1 |
| APC-Res.pl.-2 Heteroz FV-Leiden | 34,0 | 61,0 | 24,0 |
| APC-Res.pl.-3 Homoz FV-Leiden | 39,8 | 44,0 | 22,5 |
| Kontrollplasma AK-P (OAK-Plasma) | 47,8 | 356,6 | 33,2 |
| Normal-Kontroll-Plasma | 37,2 | 91,7 | 40,9 |
| Normalbereich (MW + 2SD) | | | 30 - 60 |

Die erhaltenen Ergebnisse zeigen deutlich, daß die erfindungsgemäße Methode C auch für Plasmen mit erhöhten Basis-aPTT, gemessen mit der Methode A, verwendbar ist. Die einzigen Ausnahmen sind - verständlicherweise aufgrund der Rolle dieser Proteine bei der Blutgerinnung - Faktor V- und Faktor VIII-Mangelplasmen, wobei je eine von den APC-Substraten komplett ausgefallen ist. Eine graphische Darstellung der erhaltenen Ergebnisse ist in Fig. 1 gezeigt, wobei die horizontale Linie der "cut-off"-Wert (30 sek) darstellt, also die untere Grenze des Normalbereiches.

Eine Störung im Protein C-Pathway führt zu einer signifikanten Verlängerung der aPTT-Werte. Werte unter dem Normalbereich deuten auf abnorme Werte hin. Ein Wert weit oberhalb des Normalbereiches zeigt eine Störung im intrinsischen Blutgerinnungssystem an und zwar einen Mangel der Faktoren XII, XI, VIII, V oder Fibrinogen.

Die gezeigten Ergebnisse belegen, daß mit der erfindungsgemäßen Methode C im gezeigten Beispiel alle Plasmen, außer Faktor VIII- und Faktor V-Mangelplasmen (mit einer Faktor VIII- bzw. Faktor V-Aktivität von < 0,01 IE/ml), durch den erhaltenen Endpunkt evaluierbar sind. Mit der Vergleichsmethode B haben alle Plasmen mit erhöhter Basis-aPTT gemäß Verfahren A (Faktor II-, Faktor V-, Faktor VIII-, Faktor IX- und Faktor X-Mangelplasmen sowie die oral antikoagulierten Plasmen (OAK-Plasmen) eine sehr hohe oder überhaupt nicht meßbare aPTT.

Mit der erfindungsgemäßen Methode C liegen auch die aPTT-Werte von OAK-Plasmen im Normalbereich, niedrige Werte sind nur beim Faktor V-Leiden und bei Protein S-Mangelplasmen (wobei einer der APC-Cofaktoren fehlt oder nicht funktionsfähig ist) gemessen worden.

### Beispiel 3 : Verwendung des Reagens zur Erkennung von Mangelzuständen der Cofaktoren des Protein C-Pathways:

Folgende Proben wurden untersucht: Plasmen von gesunden Personen, APC-resistente Plasmen (Faktor V-Leiden heterozygot), Plasmen mit Protein S-Mangel und Plasmen mit Protein C-Mangel. Die Proben wurden entweder in einem Faktor V-Mangelplasma, in einem Protein S-Mangelplasma oder in einem kombinierten Faktor V-Protein S-Mangelplasma im Verhältnis 1 : 5 verdünnt. Zu 100 µl der in den verschiedenen Mangelplasmen verdünnten Plasmaproben wurden 100 µl des erfindungsgemäßen Reagens zupipettiert. Die Mischung wurde 2 min bei 37°C inkubiert und anschließend 100 µl einer 25 mM Calciumchloridlösung zum Starten der Reaktion hinzugefügt. Die Gerinnungszeit wurde in einem Kugelkoagulometer gemessen. Die erhaltenen Gerinnungszeitwerte sind in Tabelle II angeführt.

**TABELLE II**

| Test am KC 10 | | | |
|---|---|---|---|
| Proben: | FV-MP Protein S-MP Protein S-FV-MP | | |
| | Werte in Sekunden | | |
| Normalplasma | 107,7 | 82,9 | 62,5 |
| Normalplasma | 98,1 | 77,6 | 57,4 |
| low APC-Resp.Plasma | 73 | 73,7 | 50,2 |
| low APC-Resp.Plasma | 72,7 | 69,9 | 46,1 |
| Protein S-Mangelplasma | 106,7 | 61,5 | 54,9 |
| Protein C-Mangelplasma | 106,5 | 82,5 | 61,6 |

Die Ergebnisse demonstrieren, daß, wenn eine Probe im Faktor V-Mangelplasma verdünnt wird, die APC-Resistenz durch gegenüber den Normalplasmen geringere Sekundenwerte angezeigt wird. Dies ist darauf zurückzuführen, daß Faktor V-Leiden ein schlechterer Cofaktor für APC ist als Faktor V-Wildtyp. Ein Protein S-Mangel wird hingegen mit einem Faktor V-Mangelplasma nicht erkannt.

Wird eine Probe im Protein S-Mangelplasma verdünnt, wird Protein S-Mangel erkannt. APC-Resistenz wird dabei teilweise miterfaßt.

Wird eine Probe in einem kombinierten Faktor V-Protein S-Mangelplasma verdünnt, können Abnormitäten (also Mangelzustände) der beiden Cofaktoren Faktor V und Protein S sicher erkannt werden. In keinem dieser drei Fälle wird jedoch im Rahmen des vorliegenden Beispiels ein Protein C-Mangel angezeigt.

## Patentansprüche

1. Reagens zur Bestimmung der aPTT umfassend zumindest ein Gerinnungsprotein, Phospholipide und einen Aktivator der intrinsischen Gerinnung in co-lyophilisierter Form.

2. Reagens nach Anspruch 1, **dadurch gekennzeichnet, daß** das Gerinnungsprotein ausgewählt ist aus der Gruppe Vitamin K-abhängiger Faktor, aktivierter Vitamin K-abhängiger Faktor, Faktor der intrinsischen Gerinnung, aktivierter Faktor der intrinsischen Gerinnung, Prothrombinkomplex-Faktor, intrinsischer Kontaktfaktor und Cofaktor der Gerinnung.

3. Reagens nach Anspruch 1 oder 2, **dadurch gekennzeichnet, daß** das Gerinnungsprotein ausgewählt ist aus der Gruppe Faktor II, Faktor VII, Faktor IX, Faktor X, Protein C, Protein Z und Protein S, gegebenenfalls in aktivierter Form, oder eine Kombination davon ist.

4. Reagens nach Anspruch 1 oder 2, **dadurch gekennzeichnet, daß** das Gerinnungsprotein Faktor XII und/oder XI, gegebenenfalls in aktivierter Form, ist.

5. Reagens nach Anspruch 1 oder 2, **dadurch gekennzeichnet, daß** das Gerinnungsprotein Faktor V und/oder VIII ist.

6. Reagens nach einem der Ansprüche 1 bis 5, **dadurch gekennzeichnet, daß** das Gerinnungsprotein in gereinigter Form vorliegt.

7. Reagens nach einem der Ansprüche 1 bis 6, **dadurch gekennzeichnet, daß** das Gerinnungsprotein aktiviertes Protein C ist, welches in einer Aktivität von mindestens 0,05 E/ml Reagens vorliegt, vorzugsweise in einem Bereich zwischen 0,05 bis 2 E/ml, mehr bevorzugt 0,1 bis 1 E/ml, am meisten bevorzugt 0,2 bis 0,6 E/ml.

8. Reagens nach einen der Ansprüche 1 bis 7, **dadurch gekennzeichnet, daß** aktiviertes Protein C und Faktor II, IX und X sowie gegebenenfalls Faktor VII und/oder Protein S enthalten ist.

9. Reagens nach einem der Ansprüche 1 bis 8, **dadurch gekennzeichnet, daß** das Gerinnungsprotein in einer standardisierten Mischung bestehend aus Faktor II, Faktor IX und Faktor X in Konzentrationen von jeweils 0,05 bis 2 E/ml Reagens enthalten ist.

10. Reagens nach Anspruch 9, **dadurch gekennzeichnet, daß** weiters Faktor VII und/oder Protein S, vorzugsweise in einer Konzentration zwischen 0,1 bis 2 E/ml, mehr bevorzugt zwischen 0,2 bis 1 E/ml, enthalten ist.

11. Reagens nach einem der Ansprüche 1 bis 10, **dadurch gekennzeichnet, daß** der Aktivator ausgewählt ist aus der Gruppe Ellagsäure, Celit, Silizium-hältige Verbindung und Sulfatid oder ein Derivat bzw. eine Kombination davon ist.

12. Reagens nach einem der Ansprüche 1 bis 11, **dadurch gekennzeichnet, daß** die Phospholipide in reiner Form in einer standardisierten Mischung vorliegen.

13. Reagens nach einem der Ansprüche 1 bis 12, **dadurch gekennzeichnet, daß** die Phospholipide bezogen auf den Gesamtphospholipidgehalt in einer Mischung von 5 bis 50 % Phosphatidylserin, 15 bis 60 % Phosphatidylcholin, 15 bis 60 % Phosphatidylethanolamin, 0 bis 20 % Cholesterin und 5 bis 20 % Phosphatidylsäure vorliegen.

14. Reagens nach einem der Ansprüche 1 bis 13, **dadurch gekennzeichnet, daß** es frei von Calcium-Ionen ist.

15. Reagens nach einem der Ansprüche 1 bis 14, **dadurch gekennzeichnet, daß** eine Heparin-neutralisierende Substanz ausgewählt aus der Gruppe Polybren, Protamin, Salz und Heparinase enthalten ist.

16. Reagens nach einem der Ansprüche 1 bis 15, **dadurch gekennzeichnet, daß** ein Stabilisator enthalten ist, der vorzugsweise ausgewählt ist aus der Gruppe Albumin, Puffersubstanz, Aminosäure, Zucker und Gelatine oder eine Kombination davon ist.

17. Reagens nach einem der Ansprüche 1 bis 16, **dadurch gekennzeichnet, daß** ein chromogenes Substrat enthalten ist.

18. Reagens nach Anspruch 17, **dadurch gekennzeichnet, daß** weiters eine Clotbildungs-hemmende Substanz, vorzugsweise ein Tetrapeptid, enthalten ist.

19. Reagens nach einem der Ansprüche 1 bis 18, **dadurch gekennzeichnet, daß** es nach Rekonstitution als Lösung bei Raumtemperatur über einen Zeitraum von mindestens 3 Stunden, vorzugsweise mindestens 8 Stunden, am meisten bevorzugt mindestens 24 Stunden, stabil ist.

20. Verfahren zur Bestimmung der APC-Resistenz, der APC-Cofaktor-Aktivität bzw. eines APC-Substrates in einer Probe, **dadurch gekennzeichnet, daß** die Probe mit einem rekonstituierten Reagens nach Anspruch 1 in Gegenwart von Calciumionen in Kontakt gebracht, die Blutgerinnung ausgelöst und das Ausmaß der Gerinnung bestimmt wird.

21. Verfahren nach Anspruch 20, **dadurch gekennzeichnet, daß** die APC-Cofaktor-Aktivität des Faktor V und/oder des Protein S bestimmt wird.

22. Verfahren nach Anspruch 20 oder 21, **dadurch gekennzeichnet, daß** ein Mangel und/oder eine funktionelle Störung eines APC-Cofaktors oder eines APC-Substrats bestimmt wird.

23. Verfahren nach einem der Ansprüche 20 bis 22, **dadurch gekennzeichnet, daß** das Vorliegen einer Faktor VIII-Anomalie qualitativ bzw. quantitativ bestimmt wird.

24. Verfahren nach einem der Ansprüche 20 bis 23, **dadurch gekennzeichnet, daß** der Probe ein Reagens umfassend Faktor II, IX und X sowie gegebenenfalls Faktor VII und/oder Protein S zugegeben wird.

25. Verfahren nach einem der Ansprüche 20 bis 24, **dadurch gekennzeichnet, daß** der Probe ein Faktor V-Mangelplasma, Protein S-Mangelplasma oder Faktor V/Protein S-Mangelplasma zugegeben wird.

26. Verfahren nach einem der Ansprüche 20 bis 25, **dadurch gekennzeichnet, daß** das Ausmaß der Gerinnung über die Gerinnungszeit, insbesondere unter Verwendung eines Koagulometers, bestimmt wird.

27. Verfahren nach einem der Ansprüche 20 bis 26, **dadurch gekennzeichnet, daß** ein Normalbereich für den zu bestimmenden APC-Cofaktor oder für das APC-Substrat ausschließlich in Anwesenheit von APC ermittelt wird.

28. Verwendung des Reagens nach Anspruch 1 zur Bestimmung eines Mangels und/oder einer funktionellen Störung der Protein C-Pathways, insbesondere der APC-Resistenz, eines APC-Cofaktors oder eines APC-Substrats und/oder zur Bestimmung einer Faktor VIII-Anomalie.

29. Set zur Bestimmung der aPTT enthaltend
(i) ein Reagens gemäß Anspruch 1 und
(ii) eine Kontrolle bzw. einen Kalibrator.

## Claims

1. A reagent for determining the aPTT, comprising at least one coagulation protein, phospholipids and an activator of intrinsic coagulation in co-lyophilized form.

2. A reagent according to claim 1, **characterised in that** the coagulation protein is selected from the group of vitamin K-dependent factor, activated vitamin K-dependent factor, factor of intrinsic coagulation, activated factor of intrinsic coagulation, prothrombin complex factor, intrinsic contact factor and co-factor of coagulation.

3. A reagent according to claim 1 or 2, **characterised in that** the coagulation protein is selected from the group of factor II, factor VII, factor IX, factor X, protein C, protein Z and protein S, optionally in activated form, or a combination thereof.

4. A reagent according to claim 1 or 2, **characterised in that** the coagulation protein is factor XII and/or factor XI, optionally in activated form.

5. A reagent according to claim 1 or 2, **characterised in that** the coagulation protein is factor V and/or factor VIII.

6. A reagent according to any one of claims 1 to 5, **characterised in that** the coagulation protein is provided in purified form.

7. A reagent according to any one of claims 1 to 6, **characterised in that** the coagulation protein is activated protein C which is provided in an activity of at least 0.05 U/ml of reagent, preferably in a range of between 0.05 to 2 U/ml, more preferred 0.1 to 1 U/ml, most preferred 0.2 to 0.6 U/ml.

8. A reagent according to any one of claims 1 to 7, **characterised in that** activated protein C and factors II, IX and X as well as optionally factor VII and/or protein S are contained.

9. A reagent according to any one of claims 1 to 8, **characterised in that** the coagulation protein is contained in a standardised mixture comprised of factor II, factor IX and factor X in concentrations of from 0.05 to 2 U/ml of reagent each.

10. A reagent according to claim 9, **characterised in that** furthermore factor VII and/or protein S, preferably at a concentration of between 0.1 to 2 U/ml, more preferred between 0.2 to 1 U/ml, is contained.

11. A reagent according to any one of claims 1 to 10, **characterised in that** the activator is selected from the group ellagic acid, celite, silicon-containing compound and sulfatide or a derivative or combination thereof, respectively.

12. A reagent according to any one of claims 1 to 11, **characterised in that** the phospholipids are provided in pure form in a standardised mixture.

13. A reagent according to any one of claims 1 to 12, **characterised in that** the phospholipids, based on the total phospholipid content, are provided in a mixture of from 5 to 50% phosphatidyl serine, 15 to 60% phosphatidyl choline, 15 to 60% phosphatidyl ethanolamine, 0 to 20% cholesterol and 5 to 20% phosphatidylic acid.

14. A reagent according to any one of claims 1 to 13, **characterised in that** it is free from calcium ions.

15. A reagent according to any one of claims 1 to 14, **characterised in that** a heparin-neutralising substance selected from the group polybren, protamine, salt and heparinase is contained.

16. A reagent according to any one of claims 1 to 15, **characterised in that** a stabiliser is contained which preferably is selected from the group of albumin, buffer substance, amino acid, sugar and gelatin, or a combination thereof.

17. A reagent according to any one of claims 1 to 16, **characterised in that** a chromogenic substrate is contained therein.

18. A reagent according to claim 17, **characterised in that** furthermore a clot-formation-inhibiting substance, preferably a tetrapeptide, is contained therein.

19. A reagent according to any one of claims 1 to 18, **characterised in that** it is stable upon reconstitution as a solution at room temperature for a period of time of at least 3 hours, preferably at least 8 hours, most preferred at least 24 hours.

20. A method for determining the APC resistance, the APC co-factor activity, or an APC substrate, respectively, in a sample, **characterised in that** the sample is contacted with a reconstituting reagent according to claim 1 in the presence of calcium ions, the blood coagulation is triggered, and the amount of coagulation is determined.

21. A method according to claim 20, **characterised in that** the APC co-factor activity of factor V and/or of protein S is determined.

22. A method according to claim 20 or 21, **characterised in that** a deficiency and/or a dysfunction of an APC co-factor or an APC substrate is determined.

23. A method according to any one of claims 20 to 22, **characterised in that** the presence of a factor VIII abnormality is determined qualitatively or quantitatively, respectively.

24. A method according to any one of claims 20 to 23, **characterised in that** a reagent comprising factors II, IX and X as well as, optionally, factor VII and/or protein S is added to the sample.

25. A method according to any one of claims 20 to 24, **characterised in that** a factor V deficiency plasma, protein S deficiency plasma or factor V/protein S deficiency plasma is added to the sample.

26. A method according to any one of claims 20 to 25, **characterised in that** the amount of the coagulation is determined via the coagulation time, in particular by using a coagulometer.

27. A method according to any one of claims 20 to 26, **characterised in that** a normal range for the APC co-factor to be determined or for the APC substance is exclusively determined in the presence of APC.

28. The use of the reagent according to claim 1 for determining a deficiency and/or a dysfunction of the protein C pathway, in particular the APC resistance, an APC co-factor or an APC substrate and/or for determining a factor VIII abnormality.

29. A set for determining the aPTT, comprising
(i) a reagent according to claim 1 and
(ii) a control, or a calibrator, respectively.

## Revendications

1. Réactif pour déterminer le temps de céphaline active comprenant au moins une protéine coagulante, des phospholipides et un activateur de coagulation intrinsèque dans sa forme colyophilisée.

2. Réactif selon la revendication 1, **caractérisé en ce que** la protéine coagulante est choisie dans le groupe facteur dépendant de la vitamine K, facteur activé dépendant de la vitamine K, facteur de coagulation intrinsèque, facteur activé de coagulation intrinsèque, facteur complexe prothrombine, facteur intrinsèque de contact et cofacteur de coagulation.

3. Réactif selon les revendications 1 ou 2, **caractérisé en ce que** la protéine coagulante est choisie dans le groupe facteur II, facteur VII, facteur IX, facteur X, protéine C, protéine Z et protéine S, le cas échéant dans leur forme activée, ou une combinaison de ceux-ci.

4. Réactif selon les revendications 1 ou 2, **caractérisé en ce que** la protéine coagulante est un facteur XII et/ou XI, le cas échéant dans sa forme activée.

5. Réactif selon les revendications 1 ou 2, **caractérisé en ce que** la protéine coagulante est un facteur V et/ou VIII.

6. Réactif selon l'une quelconque des revendications 1 à 5, **caractérisé en ce que** la protéine de coagulation est présente sous sa forme purifiée.

7. Réactif selon l'une quelconque des revendications 1 à 6, **caractérisé en ce que** la protéine de coagulation est la protéine C activée, laquelle est présente dans une activité d'au moins 0,05 u/ml de réactif, de préférence dans un intervalle de 0,05 à 2 u/ml, voire de 0,1 à 1 u/ml, ou mieux de 0,2 à 0,6 u/ml.

8. Réactif selon l'une quelconque des revendications 1 à 7, **caractérisé en ce qu'**il contient la protéine C activée et le facteur II, IX et X, ainsi que le cas échéant le facteur VII et/ou la protéine S.

9. Réactif selon l'une quelconque des revendications 1 à 8, **caractérisé en ce que** la protéine coagulante est contenue dans un mélange standardisé constitué du facteur II, du facteur IX et du facteur X dans une concentration respective de 0,05 à 2 u/ml de réactif.

10. Réactif selon la revendication 9, **caractérisé en ce qu'**il contient en plus le facteur VII et/ou la protéine S, de préférence dans une concentration comprise entre 0,1 et 2 u/ml, voire mieux entre 0,2 et 1 u/ml.

11. Réactif selon l'une quelconque des revendications 1 à 10, **caractérisé en ce que** l'activateur est choisi dans le groupe acide ellagique, célite, composé contenant du silicium et sulfatide, ou est un dérivé, respectivement une combinaison de ceux-ci.

12. Réactif selon l'une quelconque de revendications 1 à 11, **caractérisé en ce que** les phospholipides sont présents sous leur forme pure dans un mélange standardisé.

13. Réactif selon l'une quelconque des revendications 1 à 12, **caractérisé en ce que** les phospholipides rapportés à la teneur globale en phospholipides sont présents dans un mélange de 5 à 50 % de phosphatidylsérine 15 à 60 % de phosphatidylcholine, 15 à 60 % de phosphatidyléthanolamine, 0 à 20 % de cholestérine et 5 à 20 % d'acide phosphatidylique.

14. Réactif selon l'une quelconque des revendications 1 à 13, **caractérisé en ce qu'**il est dépourvu d'ions calcium.

15. Réactif selon l'une quelconque des revendications 1 à 14, **caractérisé en ce qu'**il contient une substance neutralisant l'héparine choisie dans le groupe polybrène, protamine, sel et héparinase.

16. Réactif selon l'une quelconque des revendications 1 à 15, **caractérisé en ce qu'**il contient un stabilisant qui est de préférence choisi dans le groupe albumine, substance tampon, acides aminés, sucre et gélatine, ou est une combinaison de ceux-ci.

17. Réactif selon l'une quelconque des revendications 1 à 16, **caractérisé en ce qu'**il contient un substrat chromogène.

18. Réactif selon la revendication 17, **caractérisé en ce qu'**il contient en outre une substance arrêtant la coagulation, de préférence un tétrapeptide.

19. Réactif selon l'une quelconque des revendications 1 à 18, **caractérisé en ce qu'**il est stable après reconstitution en solution à température ambiante pendant une durée d'au moins 3 heures, de préférence au moins 6 heures, voire au moins 24 heures.

20. Procédé pour déterminer la résistance APC, l'activité de cofacteur APC, respectivement un substrat APC dans un échantillon, **caractérisé en ce que** l'échantillon est mis en contact avec un réactif reconstitué selon la revendication 1 en présence d'ions calcium, **en ce que** la coagulation sanguine est déclenchée et **en ce qu'**on détermine l'étendue de la coagulation.

21. Procédé selon la revendication 20, **caractérisé en ce qu'**on détermine l'activité de cofacteur APC du facteur V et/ou de la protéine S.

22. Procédé selon les revendications 20 ou 21, **caractérisé en ce qu'**on détermine une carence et/ou un trouble fonctionnel d'un cofacteur APC ou d'un substrat APC.

23. Procédé selon l'une quelconque des revendications 20 à 22, **caractérisé en ce qu'**on détermine la présence d'une anomalie qualitative, respectivement quantitative du facteur VIII.

24. Procédé selon l'une quelconque des revendications 20 à 23, **caractérisé en ce que** l'échantillon est adjoint d'un réactif comprenant les facteurs II, IX et X, ainsi que le cas échéant le facteur VII et/ou la protéine S.

25. Procédé selon l'une quelconque des revendications 20 à 24, **caractérisé en ce qu'**on ajoute à l'échantillon un plasma appauvri en facteur V, un plasma appauvri en protéine S, ou un plasma appauvri en facteur V / protéine S.

26. Procédé selon l'une quelconque des revendications 20 à 25, **caractérisé en ce qu'**on détermine l'étendue de la coagulation d'après le temps de coagulation, en particulier par l'utilisation d'un coagulomètre.

27. Procédé selon l'une quelconque des revendications 20 à 26, **caractérisé en ce que** l'on recherche un écart normal pour le cofacteur APC à déterminer ou pour le substrat APC exclusivement en la présence d'APC.

28. Utilisation du réactif selon la revendication 1 pour déterminer une carence et/ou un trouble fonctionnel des cheminements de la protéine C, en particulier de la résistance APC, d'un cofacteur APC ou d'un substrat APC et/ou pour déterminer une anomalie du facteur VIII.

29. Matériel pour déterminer le temps de céphaline active comprenant
(i) un réactif selon la revendication 1 et
(ii) un contrôle, respectivement un calibreur.
